# EUROPEAN PATENT APPLICATION

(11) **EP 1 980 250 A1**
(43) Date of publication of application: **15.10.2008**
(21) Application number: 07704740.5
(22) Date of filing: 23.01.2007
(51) Int. Cl.: A61K 31/4412, A61P 17/06

(54) **USE OF MIMOSINE OR A DERIVATIVE THEREOF FOR TREATING THE CUTANEOUS EFFECTS OF PSORIASIS AND RELATED SKIN DISORDERS, AND COSMETIC OR PHARMACEUTICAL COMPOSITION CONTAINING SAME**

(30) Priority: 23.01.2006 AR P060100235
(71) Applicant: XL Global Corporation, Road Town (VG)
(72) Inventor: GABRIEL ZEITUNE, Moises, (1426) Ciudad Autonoma de Buenos Aires (AR); MARTIN LAGUENS, Rubén, (1896) Prov. Bs.As. (AR); PARENTE DUEÑA, Antonio, 08850 Gavá, Barcelona (ES)
(74) Representative: Carvajal y Urquijo, Isabel
(86) International application number: PCT/ES2007/000026
(87) International publication number: WO 2007/082974

(57) **Abstract**

The present invention relates to the use of mimosine or a derivative thereof for treating the cutaneous effects of psoriasis and related skin disorders.

## Description

The present invention relates to the use of mimosine or a derivative thereof for treating the cutaneous effects of psoriasis and related skin disorders. In a particular embodiment of the invention, mimosine is used in combination with idebenone or a derivative thereof.

Also within the scope of the invention is a composition for treating the cutaneous effects of psoriasis and related skin disorders, applicable on the skin, comprising mimosine or an acceptable derivative thereof. In a particular embodiment of the invention, the composition comprises mimosine or a derivative thereof in combination with idebenone or its derivatives.

The topical application of a pharmaceutical or cosmetic composition according to the invention confers a beneficial effect in cutaneous psoriasis processes and related skin disorders.

### Background of the Invention

Psoriasis is a skin disease characterized by its chronic recurring nature and its variable clinical effects. Psoriasis lesions are classified as erythematosquamous, a term indicating the simultaneous compromise of vascularization (erythema) and the epidermis (excessive formation of squamae).

Psoriasis is a disease with a universal distribution. It affects approximately 1% of the population, without gender predominance, with large variations which can depend on racial, geographic and environmental factors. The establishment of psoriasis represents a life-long threat. Most patients develop the first psoriasis plaques during the third decade of life, although cases in children of a few months of age and in extremely elderly patients have been reported. The earlier the onset of psoriasis, the more serious the symptoms tend to be over time.

The skin lesions of psoriasis are clinically characteristic, presenting as predominant features having clearly defined edges, having a surface formed by non-cohesive silver squamae, having a shiny erythema below the squamae and having Auspitz's sign. This sign is observed when the hyperkeratotic squamae are removed from a psoriatic plaque by means of mechanical scraping. In the course of a few seconds after the mechanical removal of the squama, the appearance of small drops of blood on the erythematous surface is observed. In addition to this sign, the so-called Koebner phenomenon can be observed in 20% of the patients: development of new psoriatic lesions as a result of a non-specific skin irritation.

Nails are also affected in psoriasis and there are systemic associations with other diseases, such as inflammatory intestinal disease, obstructive vascular disease and arthritis.

The most frequent clinical skin presentation pattern is that of chronic stationary psoriasis or psoriasis vulgaris, in which the erythematosquamous lesions persist for months or even years; this disease mainly affects the elbows, the knees, the scalp, the lumbar area, the umbilical region and especially behind the auricles. The plaques can extend and tend towards generalization.

Another clinical form of this condition is eruptive or guttate psoriasis. It starts as small lesions close to 1 cm in diameter in the trunk and extremities. The generalization to all the skin of a psoriasis results in the clinical variant known as psoriatic erythroderma. In other cases, inflammatory clinical symptoms predominate, with the formation of pustules with a generalized or localized distribution, the latter generally being located in palms and soles.

Naturally, the disease starts as symmetric plaques in a body region, but persists throughout life, being symptomatologically expressed in an unpredictable manner, being able to present extremely prolonged remission periods between one episode and the next. The onset of skin lesions in patients with psoriatic characteristics, or the exacerbation of pre-existing lesions, can be triggered by several external factors, such as physical traumas (Koebner phenomenon), by infections -especially upper respiratory tract infections-, stress and certain types of drugs.

Histologically, morphological changes characteristic of psoriasis are observed in most patients affected by this disease. Common clinical forms characteristically have an epidermis in which the thickness is from three to five times greater than normal. This increase of thickness is due to a higher keratinocyte production, therefore mitotic figures are frequently observed, the offer of cells to be keratinized increases and the lesion presents hyperkeratosis. This keratinization is pathological, having still nucleated cells in the uppermost strata of the epidermis, forming what is known as parakeratosis. The dermis has prominent elongated thin papilla. These papillae contain tortuous capillaries immersed in an edematous papillary stroma. Furthermore, a moderate inflammatory infiltrate is observed around blood vessels, formed by lymphocytes, macrophages, neutrophils and an increased amount of mastocytes. This infiltrate is limited to the papillary dermis. Collections of polymorphonuclear leukocytes are concomitantly observed which sometimes extend from the ends of the dermal papillae to the inside of the epidermis, where they are associated with focal spongiosis. Skin changes result in Munro's microabscesses, formed by the invasive leukocytes.

Psoriasis is a unique entity to the extent that it represents an excessive but controlled inflammation and cell proliferation process, both alterations occurring in a small space of skin. The pathogenesis is unknown, but it is known that certain factors have an important effect on the disease: the role of immune mechanisms is reflected in the large amount of activated T cells inside the affected epidermis and dermis, the presence of macrophages and the relationship between psoriasis and a certain type of class I histocompatibility antigens. Secondly, the predominant changes consist of a significant and persistent increase of keratinocyte proliferation simultaneously to a characteristic inflammatory pattern. A characteristic feature of skin affected by psoriasis is epidermal cell (keratinocyte) hyperproliferation.

In skin affected with psoriasis, the epidermal cell cycle is about 8.6 times shorter than normal (36 hours versus 311 hours). Furthermore, the cell population proliferation is two times higher, whereas 100% of the germ cells of the epidermis enter the growth fraction, compared to the 60 to 70% which enter in normal subjects. These abnormalities result in a hyperplastic epidermis generating 35,000 cells/mm²/day in a proliferative compartment containing approximately 52,000 cells/mm² of skin surface. Normal skin produces only 1,218 cells/mm²/day from a proliferative compartment containing 26,000 cells/mm². It is simultaneously observed that the keratinocytes are larger in size and the cohesion between them is lost. These changes are directly responsible for the important desquamation characteristic of psoriasis. It has been suggested that the deregulated expression of oncogenes or even a mutation in them could be the molecular factors underlying this epidermal hyperproliferation, given that these genes play a predominant role in controlling cell growth and differentiation. However, it has been demonstrated that these oncogenes are not involved in the pathogenesis of psoriasis. Nevertheless, it was demonstrated that the transforming growth factor alpha, a genetically regulated protein acting as a stimulus for keratinocyte proliferation, is overexpressed in psoriatic epidermis.

The overexpression of this growth factor would in turn be mediated by a continuous stimulation caused by chemical mediators such as the cytokines produced by the immune system, the complement cleavage products and the lipid mediators derived from arachidonic acid, such as leukotrienes. Cytokines are polypeptide mediators produced by a wide variety of cells, such as lymphocytes, macrophages, accessory cells of immune response, fibroblasts and endothelial cells, all of them present in psoriatic skin. These cytokines are stimulators of the immune system itself and furthermore regulate several functions of other cell types, such as epithelial cells. Other cytokines are antagonistic, inhibiting these functions. In psoriasis, interleukin-1 alpha is reduced, whereas interleukin-1 beta is increased, fulfilling an important role in the recruitment of new immune cells and in keratinocyte proliferation. Interleukin-8 plays a similar role, like interleukin-6. Interleukin-8 furthermore stimulates granulocytes to produce leukotrienes. The complement cleavage products are peptide structures recruiting inflammatory cells. Leukotrienes, including leukotriene B4, are chemotactic factors for neutrophils and are raised in psoriatic lesions. The leukotriene-generating enzyme, 5-lipoxygenase, is considerably increased in psoriasis lesions, whereas the inhibitors of said enzyme, such as benoxaprofen and lonapalene for example, improve the psoriasis symptoms. However, the injection of leukotriene B4 into the healthy skin of psoriatic patients does not develop the lesion. This seems to confirm the idea that the system of mediators is extremely complex and self-regulated, being that a single mediator cannot produce the chemical response to stimulate transforming epidermal growth factor production, the harmonious interaction of several of these cell function inhibitors, stimulator and mediators being needed.

In summary, the pathogenetic mechanism is extremely complex, although the primary mechanism initiating the cascade of events which in short lead to epidermal proliferation is still not known. It is likely that a minimum stimulus, which is still not known for certain, or even an environmental or bacterial antigen, acting in a genetically susceptible individual, appears in the psoriatic area and is trapped by cells residing in the epidermis with antigen-presenting capacity, such as Langerhans's cells. These cells, in addition to presenting the antigen, secrete cytokines recruiting immune system cells to the anatomic site. These immunologically competent populations in turn produce cytokines, which prolong the immune response and amplify it, recruiting the same types and other cell populations such as for example granulocytes, which synthesize other chemical mediators such as leukotrienes and activate the complement, thus closing the circuit. The local increase of these mediators leads to a higher expression of the transforming epidermal growth factor alpha, which promotes mitotic division of the keratinocytes. The increase in the epidermal replacement rate causes the hyperplasia characterizing psoriasis. It likewise causes a higher offer of keratinocytes to be desquamated, which do not mature completely and lead to parakeratosis, which characterizes the microscopic lesion and causes the clinically observed clear sign of desquamation. Cytokines with proinflammatory properties cause the vascular changes motivating erythema, which completes the clinical erythematosquamous lesion symptoms.

Different topical skin compositions containing one or more ingredients which can reduce epidermal cell proliferation and the inflammatory symptoms characterizing psoriasis have been described to date. One of them is based on anthralin derivatives, such as for example anthralin, which is a potent reducing agent causing an irritative dermatitis when it is used in excess, staining the surrounding skin and clothes. Tar is also used for the same purpose, as it has demonstrated a relative efficacy and does not have major collateral complications. Topical corticosteroids have also been used to reduce inflammation, with different results. A synthetic form of vitamin D3, approved for topical use and which can be used for treating nail and scalp psoriasis has also been described, which reduces the rate of growth of skin cells and removes psoriatic plaques.

Phototherapy has recently also been incorporated to the therapeutic collection against psoriasis, since it has been described that the ultraviolet light from the sun improves clinical psoriasis symptoms. The mechanism of action would be related to the pathogenesis of psoriasis, since it is known that UV light reduces the number of epidermal Langherhans' cells. Methodologically, the phototherapy for psoriasis must be accompanied by the ingestion of a sensitizing agent such as psoralens, followed by a variable ultraviolet radiation for each patient. Clinical improvements are observed shortly after 19 to 25 sessions, therefore this therapy has serious use limitations. Psoralens in turn bind covalently to cell DNA and have a certain degree of systemic toxicity.

Systemic treatments for psoriasis which are generally reserved for cases refractory to topical treatments are known, as well as combined treatments between the different therapeutic types, limited to certain particular cases. Corticosteroids must be mentioned among the systemic treatments, the administration of which is based on their anti-inflammatory effect. Their use is not only associated to a number of undesirable effects, but also psoriasis tends to reappear, even in a more serious form, upon interrupting the treatment.

Immunosuppressive drugs such as methotrexate are also used for treating psoriasis, since these compounds inhibit DNA synthesis. The risk of the treatment with methotrexate should be considered because prolonged use can lead to hepatic lesions and cirrhosis.

Other systemic treatments include cyclosporine, a potent immunosuppressant eliminating T cell populations. The results have not been encouraging and the side-effects have been high.

Etretinate is vitamin A derivative which has been used as a systemic treatment for psoriasis with good results after one week of treatment, with the disappearance of the squamae, although the erythematous lesion persists and still becomes larger. The interruption of the treatment is associated to recurrences. It is a teratogenic drug, therefore it is not indicated for all patients.

There are several plants with medicinal properties from the chemical composition of which chemical compounds with the capacity to inhibit the cell replication cycle of mammalian cells are isolated. Certain legumes, such as for example Leucaena leucoceophala, acacia and Mimosa tenuiflora (known with the common name of Tepescohuite), all of them having in their chemical composition variable amounts of the amino acid mimosine, are emphasized among them.

Mimosine (beta-N-(3-hydroxy-4-pyridone)-alpha-aminopropionic acid) is cleaved to 3-hydroxy-4(1H)-pyridone when it loses the aminopropionic ring (also known as 3,4-dihydroxypyridine or 3,4DHP). Another active metabolite is 2,3-dihydroxypyridine (2,3DHP). Both substances are toxic for mammals if they are orally administered. Mimosine is a mitosis inhibitor.

Before cells are divided into two daughter cells, they must duplicate their DNA such that each new daughter cell has the same amount of DNA as the progenitor cell. When a suitable stimulus induces the cell to enter into mitosis, specific intracellular mechanisms are started. This triggers molecular signals causing DNA duplication. This phase of the cell replication cycle, lasting a few hours, is known as the S phase (synthesis phase) of the cycle.

When the S phase ends, the cell must be indefectibly divided. This latter event is known as mitosis or M phase of the cell cycle. Between the S phase and the M phase there is a time gap, during which the cell with double DNA content prepared certain biochemical events for entering into mitosis. This gap is known as the G2 phase (G for gap) of the cell cycle, and there is obviously a G1 phase corresponding to the gap between the end of mitosis and the beginning of a new S phase, in which the cell prepares other biochemical events for entering into the DNA synthesis process. The complete cycle lasts about 30 hours in cells which are being continuously divided, but in other cell types, after mitosis, the cells can enter into a quiescent phase, called G0, during which they can be recruited if needed, in order to again start being divided, entering into a G1 phase and then into the complete cycle.

Mimosine inhibits the cell cycle during the late G1 phase and the early S phase upon interacting with a 50kDa polypeptide, p50, with 96% homology with the serine hydroxymethyltransferase enzyme. This enzyme acts during thymidylate synthesis. Thymidylic acid is a part of DNA. Other very important enzymes of the cell cycle are also reversibly inhibited by mimosine in a dose-dependent manner.

Mimosine is furthermore an iron-chelating agent and acts intracellularly as an iron sequestering agent. Due to the fact that iron is a co-factor for the ribonucleotide reductase enzyme, responsible for reducing nucleotides during DNA synthesis and therefore a key part in the cell cycle, the chelation of iron makes the latter unavailable for the correct operation of the enzyme. This is why the chelation of iron reduces mitotic rhythms.

Another effect of mimosine is the suppression of the post-translational hydroxylation of collagen precursors, therefore causing a decrease in type I and II procollagen synthesis.

In summary, mimosine can reversibly stop mitotic division, synchronizing tissue growth rhythm through well known, although complex, mechanisms in a dose-dependent manner. Furthermore, mimosine prevents programmed cell death (apoptosis) and has specific activities on collagen synthesis.

In addition, idebenone is a benzoquinone having cytoprotective effects and the pharmacodynamic properties of which have been described in patent US 4,271,083, for example. Idebenone is the generic name of the compound 6-(10-hydroxydecyl)-2,3-dimethoxy-5-methyl-1,4-benzoquinone (JP-B-62 3134 (1987), U.S. Pat. No. 4,139,545). Data obtained from *in vitro* assays suggest that the cytoprotective action of idebenone is reached upon facilitating the convergence of electrons in the mitochondrial respiratory cycle, inhibiting lipid peroxidation, reducing non-respiratory oxygen consumption and stimulating ATP formation.

Idebenone is considered to be a synthetic coenzyme Q10 and it is used, orally administered, to improve cognitive disorders, Alzheimer's disease, dementias and cerebrovascular disorders, and as a cardiac cytoprotective agent. Due to its antioxidant properties, it is administered orally, individually or in combination with other active substances, preferably with those also having antioxidant properties, such as vitamin E for example.

Patent documents DE 3,049,039, EP 0,788,793, US 4,436,753, US 5,059,627 and US 5,916,925 describe oral, parenteral or percutaneous preparations comprising idebenone or its derivatives, which can be used for treating dementia, blood circulation disorders or for inducing neural growth factors. Patent JP 1,279,818 particularly describes the use of idebenone and its derivatives in several preparations which can be used to give exogenous color to hair (idebenone is a powder having a strong orange color). Important toxic effects for idebenone have not been described to date (Arzneim. Forsch/drug res. 35 (II), 11, pg. 1704, 1985).

Patent US 6,756,045 describes the use of topical idebenone for cosmetic or dermatological uses in cases of oxidative damage of the skin, such as skin aging, wrinkles, exposure to ultraviolet light and degenerative processes.

The research of the inventors has led to the discovery of the topical use of idebenone to cause an important decrease of pigment concentration in pigmented areas of the skin without causing important side-effects (patent applications AR20040000014 and WO2005065670)

The inventors have surprisingly found that a preparation for topical cutaneous use comprising mimosine, either alone or in combination with the compound idebenone, can cause an important decrease of the skin lesion characterizing psoriasis without causing important side-effects.

### Brief Description of the Invention

The present invention relates to the use of mimosine or a derivative thereof for treating the cutaneous effects of psoriasis and related skin disorders. It preferably relates to the use of mimosine or a derivative thereof in combination with idebenone or a derivative thereof for treating the cutaneous effects of psoriasis and related skin disorders. Particularly, mimosine, idebenone and/or the acceptable derivatives thereof are found in an effective amount to produce epidermal proliferation inhibition and the clinical improvement of the skin. Also particularly, the skin disorders can be selected from the group comprising psoriasis, rosacea, photodamaged skin, atopic dermatitis, eczemas and seborrheic dermatitis.

Within the particular objects of the invention, mimosine or an acceptable derivative thereof can be used in a cosmetic composition intended to be applied on the skin, useful for producing a cosmetic improvement of psoriatic skin at the application site, or be used to prepare a dermatological composition useful for producing a clinical improvement of psoriatic skin at the application site. Mimosine or its derivatives are preferably used in combination with idebenone or its derivatives.

Another object of the invention is a composition for treating the cutaneous effects of psoriasis and related skin disorders, applicable on the skin, comprising mimosine or an acceptable derivative thereof, preferably in an amount comprised between 0.01 % and 30% w/w or v/w. The composition can particularly further comprise idebenone or an acceptable derivative thereof, preferably in an amount comprised between 0.01% and 30% w/w or v/w. In an embodiment of the invention, the composition of the invention can also comprise an extract of tepescohuite or of its acceptable derivatives.

Within the different embodiments, it is contemplated that the composition of the invention can be topically administered and is in the form of an occlusive patch, cream, gel, emulsion, spray, etc. According to the Galenic form which it is in, the composition can further comprise components selected from oily components and hydrosoluble components, such as for example self-emulsifiable wax, petroleum jelly, isopropyl myristate and cetyl alcohol, glycerol, methylparaben and propylparaben, beneficial agents for the skin, and cosmetically and/or pharmaceutically acceptable antioxidants.

In addition, within the scope of the invention, a method for treating the cutaneous effects of psoriasis and related skin disorders is provided, which comprises applying at the lesioned site a composition comprising an effective amount of mimosine or its derivatives or an effective amount of mimosine or its derivatives in combination with an effective amount of idebenone or its derivatives, allowing it to act overnight and rinsing in the morning. Likewise, a method for treating the cutaneous effects of psoriasis and related skin disorders is provided, which comprises applying at the lesioned site the composition of the invention, two times a day. According to the invention, the method of the invention can be complemented with the irradiation of the patient with ultraviolet light.

### Detailed Description of the Invention

The present invention relates to a composition intended for the topical application on the skin, comprising a cosmetically or pharmaceutically effective amount of mimosine, its derivatives or mixtures thereof, alone or combined with a cosmetically or pharmaceutically effective amount of idebenone, its derivatives, or mixtures thereof. The present invention particularly relates to a cosmetic or dermatological preparation which can confer a beneficial effect in epidermal proliferation processes characterizing cutaneous psoriasis. The present invention preferably relates to cosmetic or dermatological preparations comprising mimosine and/or its derivatives alone or combined with idebenone and/or its derivatives, and decreasing the local increase of the thickness of the epidermis, of hyperkeratosis, of parakeratosis of the skin caused by an increase in the production of a larger number of keratinocytes, and decreasing the dermal inflammatory process accompanying cutaneous psoriasis.

The present invention also relates to the use of mimosine alone or combined with idebenone in a dermatological composition intended to be applied on the skin for the purpose of producing a decrease of the kinetics of epidermal proliferation of the skin at the application site. The mimosine of the composition of the present invention can indistinctly be incorporated as an ingredient of botanical or phytoderivative extracts in pharmaceutically or cosmetologically acceptable amounts.

In this document, the terms mimosine; leucenol; leucenine; leucaenine; leucaenol; alpha-amino-3-hydroxy-4-oxo-1(4H)-pyridine propanoic acid, 3-hydroxy-4-oxo-1(4H)-pyridine alanine; beta-[N-(3-hydroxy-4-pyridone)]-alpha-aminopropionic acid, are used indistinctly as synonyms and are exchangeable.

Likewise, in this document, the term "decrease of the kinetics of epidermal proliferation" must be understood as obtaining a stop of epidermal hyperproliferation in a given area of the skin until achieving a texture and thickness similar to that of the surrounding skin. The proliferating area can particularly be due to any skin disorder and more particularly to skin disorders selected from psoriasis, rosacea, skin damaged by ultraviolet radiation, atopic dermatitis, chronic dermatitis, eczemas, prurigos and seborrheic dermatitis.

The composition intended for the topical application on the skin can be found, for example, in the form of a cream, gel, occlusive patch, emulsion or spray. The composition of the invention is preferably in the form of a (O/W) cream.

The composition intended to be applied on the skin of the invention preferably comprises idebenone or a derivative thereof in an amount comprised between 0.01% and 30% w/w and mimosine or a derivative thereof in an amount comprised between 0.01 % and 30% w/v. Still more preferably, idebenone or its derivative is in an amount comprised between 0.01% and 5% w/w and mimosine or its derivatives are in an amount comprised between 0.01% and 5%.

The composition of the invention could be formulated by a person skilled in the art using known cosmetically or pharmaceutically acceptable excipients. The composition will preferably comprise oily components and hydrosoluble components. Examples of oily components can be self-emulsifiable wax, petroleum jelly, isopropyl myristate and cetyl alcohol. Examples of hydrosoluble components can be glycerol, propylene glycol, methylparaben.

Likewise, the composition of the invention could further contain beneficial agents for the skin such as for example wetting agents, moisturizers and vitamins, which are known and can be chosen by the person skilled in the art. If necessary, the composition of the invention can further comprise cosmetically and/or pharmaceutically acceptable antioxidants.

The composition of the invention could also further contain beneficial agents for a better cutaneous absorption such as for example, the absorption promoters d-limonene, terpenes, saponins, menthol and propylene glycol, which are known and can be chosen by the person skilled in the art, and likewise the composition of the invention could contain technically useful drug ingredients for improving absorption, such as for example, liposomes, nanosomes, niosomes and cyclodextrins, which are known and can be chosen by the person skilled in the art.

The present invention further relates to a method for treating psoriasis and related disorders which comprises applying at the lesion site a composition comprising an effective dose of mimosine alone or combined with idebenone, allowing it to act overnight and rinsing in the morning, or alternatively, an effective dose of mimosine alone or combined with idebenone, two times a day.

### Examples

### Example 1

### i) Aqueous (O/W) cream formulations containing mimosine

**Table I:**

| INGREDIENTS | % BY WEIGHT | | | | | |
|---|---|---|---|---|---|---|
| Water | Q.s.f. 100 g of cream | | | | | |
| Non-ionic self-emulsifiable wax | 6.00 | 6.00 | 6.00 | 6.00 | 6.00 | 6.00 |
| Petroleum jelly | 5.00 | 5.00 | 5.00 | 5.00 | 5.00 | 5.00 |
| Glycerol | 5.00 | 5.00 | 5.00 | 5.00 | 5.00 | 5.00 |
| Isopropyl myristate | 2.00 | 2.00 | 2.00 | 2.00 | 2.00 | 2.00 |
| Cetyl alcohol | 1.50 | 1.50 | 1.50 | 1.50 | 1.50 | 1.50 |
| Methylparaben (Nipagin) | 0.20 | 0.20 | 0.20 | 0.20 | 0.20 | 0.20 |
| Propylparaben (Nipasol) | 0.10 | 0.10 | 0.10 | 0.10 | 0.10 | 0.10 |
| Mimosine | 0.004 | 0.008 | 0.012 | 0.02 | 0.1 | 0.16 |

### ii) Aqueous (O/W) cream formulations containing mimosine and idebenone (IDB).

**Table II:**

| INGREDIENTS | % BY WEIGHT | | | | | |
|---|---|---|---|---|---|---|
| Water | Q.s.f. 100 g of cream | | | | | |
| Non-ionic self-emulsifiable wax | 6.00 | 6.00 | 6.00 | 6.00 | 6.00 | 6.00 |
| Non-ionic self-emulsifiable wax | 6.00 | 6.00 | 6.00 | 6.00 | 6.00 | 6.00 |
| Petroleum jelly | 5.00 | 5.00 | 5.00 | 5.00 | 5.00 | 5.00 |
| Glycerol | 5.00 | 5.00 | 5.00 | 5.00 | 5.00 | 5.00 |
| Isopropyl myristate | 2.00 | 2.00 | 2.00 | 2.00 | 2.00 | 2.00 |
| Cetyl alcohol | 1.50 | 1.50 | 1.50 | 1.50 | 1.50 | 1.50 |
| Methylparaben (Nipagin) | 0.20 | 0.20 | 0.20 | 0.20 | 0.20 | 0.20 |
| Propylparaben (Nipasol) | 0.10 | 0.10 | 0.10 | 0.10 | 0.10 | 0.10 |
| Idebenone | 0.1 | 0.2 | 0.3 | 0.5 | 2.5 | 4 |
| Mimosine | 0.004 | 0.008 | 0.012 | 0.02 | 0.1 | 0.16 |

### iii) Aqueous (O/W) cream formulations containing extract of tepescohuite

**Table III:**

| INGREDIENTS | % BY WEIGHT | | | | | |
|---|---|---|---|---|---|---|
| Water | Q.s.f. 100 g of cream | | | | | |
| Non-ionic self-emulsifiable wax | 6.00 | 6.00 | 6.00 | 6.00 | 6.00 | 6.00 |
| Petroleum jelly | 5.00 | 5.00 | 5.00 | 5.00 | 5.00 | 5.00 |
| Glycerol | 5.00 | 5.00 | 5.00 | 5.00 | 5.00 | 5.00 |
| Isopropyl myristate | 2.00 | 2.00 | 2.00 | 2.00 | 2.00 | 2.00 |
| Cetyl alcohol | 1.50 | 1.50 | 1.50 | 1.50 | 1.50 | 1.50 |
| Methylparaben (Nipagin) | 0.20 | 0.20 | 0.20 | 0.20 | 0.20 | 0.20 |
| Propylparaben (Nipasol) | 0.10 | 0.10 | 0.10 | 0.10 | 0.10 | 0.10 |
| Extract of tepescohuite | 0.20 | 0.40 | 0.6 | 1.0 | 5 | 8 |

### iv) Aqueous (O/W) cream formulations containing extract of tepescohuite and idebenone (IDB).

**Table IV:**

| INGREDIENTS | % BY WEIGHT | | | | | |
|---|---|---|---|---|---|---|
| Water | Q.s.f. 100 g of cream | | | | | |
| Non-ionic self-emulsifiable wax | 6.00 | 6.00 | 6.00 | 6.00 | 6.00 | 6.00 |
| Petroleum jelly | 5.00 | 5.00 | 5.00 | 5.00 | 5.00 | 5.00 |
| Glycerol | 5.00 | 5.00 | 5.00 | 5.00 | 5.00 | 5.00 |
| Isopropyl myristate | 2.00 | 2.00 | 2.00 | 2.00 | 2.00 | 2.00 |
| Cetyl alcohol | 1.50 | 1.50 | 1.50 | 1.50 | 1.50 | 1.50 |
| Methylparaben (Nipagin) | 0.20 | 0.20 | 0.20 | 0.20 | 0.20 | 0.20 |
| Propylparaben (Nipasol) | 0.10 | 0.10 | 0.10 | 0.10 | 0.10 | 0.10 |
| Idebenone | 0.1 | 0.2 | 0.3 | 0.5 | 2.5 | 4 |
| Extract of tepescohuite | 0.20 | 0.40 | 0.6 | 1.0 | 5 | 8 |

### iii) Process for preparing creams containing mimosine alone or combined with idebenone or extracts of tepescohuite alone or combined with idebenone

Step 1: The oily components (A) of the formulation (self-emulsifiable wax, petroleum jelly, isopropyl myristate and cetyl alcohol) are melted and heated to a temperature of 70/75 °C. The hydrosoluble components (B) of the formulation (glycerol) are dissolved in separate in the necessary amount of water, as described above, and heated to 70/75 ° C.
Step 2: Then, controlling that the temperature of the oily and hydrosoluble components is the same, the oily phase (A) is added on the aqueous phase (B) with vigorous and constant stirring. It is cooled in a water bath and stirred slowly until the temperature reaches 55 °C and methylparaben and propylparaben are added. It is continued to be cooled in a water bath and stirred until the temperature reaches 45 °C.
Step 3: Once the desired temperature has been reached, mimosine or the extract of tepescohuite is added with slow stirring until obtaining a homogeneous preparation. It is allowed to cool until the desired consistency and packaged suitably.
Step 4: For the case of the combination of mimosine or the extract of tepescohuite with idebenone, once step 2 has been concluded and the desired temperature has been reached, the required amount of idebenone is dissolved, as described above, in 0.5 ml of ethanol and added to the previous preparation with slow stirring until obtaining a homogeneous preparation. Mimosine is then added with slow stirring until obtaining a homogeneous preparation. It is allowed to cool until the desired consistency and packaged suitably.

In the cases in which the concentration of idebenone is greater than 3%, it may be necessary to increase the proportion of the components of the oily phase until the active ingredient is solubilized. If the required concentration of idebenone is much greater than 4%, the aqueous base cream can be substituted with an oily (W/O) cream and mimosine or the extract of tepescohuite can be added.

### Example 2

### Effect of a cream with 0.1% mimosine and 2.5% idebenone on the evolution of psoriatic lesions.

For the purpose of knowing the effect of a cream containing 0.1 % mimosine and 2.5% idebenone on the evolution skin lesions of psoriasis, 10 volunteers of both sexes, of legal age, with symmetric psoriatic lesions in both elbows, with indication of topical treatment, free from treatment during the 30 days prior to the application of the cream, were selected. Once informed about the scopes and limitations of the experiment and once an informed consent was signed, all the volunteers had a prior biopsy in which the diagnosis of psoriasis vulgaris was histologically confirmed. Each of the volunteers was then indicated to apply the cream with the active ingredients 1 time a day for 28 consecutive days on the lesions of the right elbow, applied at night and allowing to act until the next morning, in the form of a generous uniform layer, gently massaging the treated area with the fingertips until the evanescence of the cream. A cream with an external appearance similar to the previous one, but lacking both active ingredients (placebo cream), was prepared and was applied on the left elbow simultaneously to the cream under study, in a similar manner, which was used as a control. Clinical evaluations were performed on day 0, 7, 14, 21 and 28, considering day 0 as the day on which the treatment started. Special attention was paid to the size of the lesioned area, to desquamation, to erythema, to itching, to superficial bleeding and to the infiltration of the psoriatic plaque, any adverse or undesirable effect which the volunteers may present likewise being recorded. The total volume of cream with active ingredients and of placebo cream was 14 grams for each of them.
Results: After 7 days, all the volunteers had substantially improved the desquamation of the plaques in both elbows in a similar manner, the size thereof, the itching and the infiltration of the psoriatic plaque being maintained, the 3 volunteers presenting bleeding maintaining it.

In relation to that observed on day 7, after 14 days, all the volunteers did not show significant changes in the left elbow, where the placebo cream used as a control had been applied. In contrast, the right elbow showed in all the volunteers less desquamation and an attenuation of erythema, with a decrease of the degree of infiltration of the plaque. The itching decreased more in the right elbow than in the left elbow. Cases of bleeding were not recorded in any of the elbows.

After 21 days, all the volunteers presented the same clinical characteristics on the control left elbow as in the prior evaluations. The right elbow showed a clear involution of the size of the lesion in 5/10 volunteers and a virtually complete flattening of the plaque in 8/10 volunteers. Signs of desquamation of the right elbow were not recorded in any case. Erythema had a lower intensity in the right elbow in relation to the left elbow in all the volunteers and they did not declare the presence of itching in the right elbow, whereas in the left elbow, itching still persisted, although with less intensity. Bleeding was not recorded in any of the volunteers in any of the elbows.

After 28 days, all the volunteers had the same clinical characteristics on the control left elbow as in the prior evaluations. The right elbow showed a clear involution of the size of the lesion in 8/10 volunteers, coinciding with a complete flattening thereof. Erythema was scarce in these 8 volunteers, but it still persisted. Two of the 10 volunteers showed a complete disappearance of the lesion of the right elbow, the latter being covered with skin with a normal appearance.

Only 1 volunteer reported a burning sensation on the right elbow upon applying the 5^{th} to 8^{th} application of the cream, which lasted in each case for a few minutes in order to later lessen, it not being necessary to suspend the treatment. The same volunteer reported a sporadic burning sensation with a very short duration, which did not reach one minute, in some other later applications, with an establishment immediately upon the application of the cream. This volunteer was one of those presenting complete remission of the lesion on the right elbow. Most of the volunteers used the 14 grams of cream which had been given to them.
Conclusions: The cream with 0.1 % mimosine and 2.5% idebenone proved to be highly effective for reducing the size of psoriatic skin lesions, moderating their clinical characteristics, even reaching the complete cure, and substantially decreasing the associated symptomatology. The apparent improvement obtained 7 days after starting the treatment, both in the treated elbow and in the control elbow can be attributed to the emollient effect of the common excipients of both creams. The cream under study proved to be safe, as there was only 1 case of a volunteer with mild secondary phenomena such as burning sensation, with a spontaneous disappearance.

### Example 3

### Effect of a cream with 0.1% mimosine and 2.5% idebenone on the histology of psoriatic lesions.

For the purpose of evaluating the morphological changes occurring with the application of a cream containing 0.1% mimosine and 2.5% idebenone, biopsies were obtained from both elbows of the volunteers of Example 2, 28 days after starting the treatment, using a punch with a diameter of 5 mm and the histological image was compared between both elbows and between them and the prior biopsies performed on the volunteers to corroborate the diagnosis of psoriasis.

The material obtained was fixed in a buffered 10% formaldehyde solution, pH 6.8, for 24 hours, dehydrated in alcohols of increasing concentration, included in paraffin, cut in a sliding microtome to a thickness of 4 um and stained with hematoxylin and eosin and with periodic acid-Schiff (PAS), according to the usual practice of histological processing. The observations were carried out with a binocular microscope. Results: All the lesions of the left elbow of all the volunteers showed characteristic lesions of psoriasis, consisting of an epidermal spinous hyperplasia, with regular acanthosis and hyperkeratosis with parakeratosis areas. The dermal papillae were elongated and infiltrated by a moderate to intense inflammatory process, with inflammatory element exocytosis towards the epidermis, the formation of intraepidermal Munro's microabscesses being observed in 6 of the volunteers. The papillary blood vessels appeared dilated and with parietal. The PAS staining showed thickening of the epidermal basement membrane in a diffuse manner, like the vascular basement membranes of the vessels of the papillary dermal plexus. The images were similar in all the studied cases and also similar to those corresponding to the biopsies performed prior to the recruitment of the volunteers.

On the contrary, all the right elbow samples of all the volunteers showed minimum non-specific changes which did not correspond to the histological pattern of a psoriasis, with the complete disappearance of parakeratosis in 8/10 cases, attenuation of hyperkeratosis in 8/10 cases and absence of hyperkeratosis in the 2 remaining cases. The epidermal thickness was clearly reduced in most cases and normalized in 3 of them. Elongations of dermal papillae or intraepidermal microabscesses or vascular disorders were not observed. The dermal inflammatory process was mild in 6/10 cases, moderate in 1 case and non-existent in the other 3 cases. With PAS staining a single case of thickening of the vascular basement membranes was observed, corresponding to the case which showed the greatest inflammation. The epidermal basement membranes were not thickened.
Conclusion: The cream with 0.1 % mimosine and 2.5% idebenone significantly reduced the morphologically evaluated psoriatic lesion, to the point of not being able to diagnose the disease and samples of virtually normal skin being observed 28 days after its application.

### Example 4

### Comparative study of a cream with 2.5% idebenone alone, with a cream with 0.1% mimosine, and with a cream with 0.1% mimosine and 2.5% idebenone for treating psoriasis vulgaris.

For the purpose of knowing the effect on psoriasis of each active ingredient of the formula separately, 10 volunteers with psoriasis vulgaris with symmetric lesions in elbows, with prior histological confirmation of their psoriatic condition, were selected. Five volunteers received a cream with 0.1% mimosine and 2.5% idebenone on their right elbow daily for 28 days, and a cream with 2.5% idebenone on their left elbow in a similar manner, and another 5 volunteers received the cream with 0.1 % mimosine and 2.5% idebenone on their right elbow and a cream with 0.1% mimosine on the left elbow, with a scheme similar to that of the other group. The clinical evolution of the lesions was evaluated 7, 14, 21 and 28 days after staring the treatment and a biopsy sample of each elbow during day 28 was obtained.
Results: Seven days after starting the treatment, the lesions of both elbows in the two groups of volunteers showed a clinical improvement characterized by a decrease o epidermal desquamation. From day 14 onwards, a clear progressive improvement was observed on the right elbow which had received the cream with 0.1% mimosine and 2.5% idebenone, which reached its maximum expression towards day 28, with a virtually complete attenuation of the lesion, in a manner similar to that described in Example 2. The 5 volunteers who were treated on their left elbow with a cream with 2.5% idebenone showed from day 14 onwards a decrease of erythema and a flattening of the indurated lesion, the psoriatic plaque being less thick. For days 21 and 28, these changes were emphasized, the size of the erythematous lesion also decreasing. Desquamation progressively diminished, but it did not disappear completely, although the squamae peeled off more cohesively than at the beginning of the experiment. The 5 volunteers who were treated on their left elbow with a cream with 0.1% mimosine showed from day 14 onwards a decrease of erythema and flattening of the lesion, although with a lower intensity than the previous group. After 21 and 28 days, the erythematous lesion and the induration were maintained in a manner similar to day 14. Superficial desquamation was no longer observed from day 14 onwards and the epidermal surface adopted a lattice similar to the normal one, although more marked, starting on day 21 and being maintained towards day 28. The scraping of the epidermal surface of these latter volunteers did not achieve peeling off squamae. 2/5 patients reported that the itching was maintained constant throughout the experiment on the left elbow, it having disappeared in the 3 remaining volunteers and in the group which received idebenone alone.

Histologically, the right elbow samples of the 10 volunteers showed a microscopic image of the skin with minimum or non-specific lesions, similar to those described in Example 3. The left elbow samples which were treated with a cream with 2.5% idebenone showed a decrease of the dermal inflammatory process, with a discrete elongation of dermal papillae and a clear decease of interstitial edema and vascular dilation. The epidermis was shown to be hyperplasic with moderate hyperkeratosis and parakeratosis foci. Intraepidermal microabscesses or important exocytosis were not observed. The PAS dye staining showed epidermal and vascular basement membranes within normal limits. The microscopic images were similar in the 5 samples evaluated. The left elbow samples which were treated with a cream with 0.1% mimosine showed a moderate dermal inflammatory process, exocytosis towards the epidermis and formation of intraepidermal Munro's microabscesses, with discrete elongation of dermal papillae, interstitial edema, vascular dilation and thickening of epidermal and vascular basement membranes, as was revealed by PAS staining. The epidermis showed a minimum epidermal hyperplasia, with discrete hyperkeratosis, without signs of parakeratosis, with an ordered keratinization. The images were similar in the 5 cases evaluated.
Conclusion: The cream with 2.5% idebenone improves the clinical appearance of the psoriatic lesion, the inflammatory component of the lesion mainly decreasing, whereas the cream with 0.1 % mimosine improves the clinical appearance of the psoriatic lesion, the proliferative epidermal component of the lesion mainly decreasing. None of the active ingredients tested separately equaled or exceeded the marked improvement obtained with the combination of both active ingredients.

### Example 5

### Effect of a cream with the 5% extract of tepescohuite and 2.5% idebenone and for treating the psoriasis vulgaris.

For the purpose of knowing if the application of a cream with the botanical extract of tepescohuite, containing mimosine in its formulation, combined with 2.5% idebenone had an action similar to a cream with the 0.1% amino acid mimosine and 2.5% idebenone, a daily dose of a cream containing 5% extract of tepescohuite and 2.5% idebenone was applied on the left elbow of 5 patients with histologically confirmed psoriasis vulgaris for 28 days, and the second cream was applied similarly on the right elbow, the clinical evaluation of both lesions being recorded after 7, 14, 21 and 28 days, and a biopsy of both elbows being obtained on day 28.
Results: In both elbows and in the 5 cases evaluated, a progressive clinical improvement, similar to that recorded in the cases described in Example 2, was observed. Histologically, the biopsies of both elbows were similar, a marked decrease of epidermal spinous hyperplasia and of hyperkeratosis, with the absence of parakeratosis, being observed. A significant flattening of the dermal papillary elongation and a clear decrease of the inflammatory process and vascular dilation were also observed, to the point that none of the samples observed under the microscope could be diagnosed as psoriasis.
Conclusion: The cream containing 0.1 % mimosine and 2.5% idebenone has a behavior very similar to the cream containing 5% extract of tepescohuite and 2.5% idebenone on the skin lesions of psoriasis vulgaris. This similarity of action can be attributed to the fact that both creams contain similar amounts of mimosine, one in a pure form and the other one with mimosine contained in the complete glycolic extract.

### Example 6

### Determination of the effect of a cream with 0.012% mimosine and 0.3% idebenone on psoriatic lesions.

For the purpose of knowing the effect of a cream containing 0.012% mimosine and 0.3% idebenone on the evolution of skin lesions of psoriasis, 5 volunteers of both sexes, of legal age, with symmetric psoriatic lesions in both elbows, free from treatment during the 30 days prior to the application of the cream were selected. All the volunteers had a prior biopsy in which the diagnosis of psoriasis vulgaris was histologically confirmed. Once informed about the scopes and limitations of the experiment and once an informed consent was signed, each of the volunteers was indicated to apply the cream with the active ingredients 1 time a day for 28 consecutive days on the lesions of the right elbow, applied at night and allowing to act until the next morning, in the form of a generous uniform layer, gently massaging the treated area with the fingertips until the evanescence of the cream. A similar cream, but lacking both active ingredients was prepared, which was applied on the left elbow simultaneously to the cream under study, in a similar manner, which was used as a control. Clinical evaluations were performed on day 0, 7, 14, 21 and 28, considering day 0 as the day on which the treatment started. Special attention was paid to the size of the lesioned area, to desquamation, to erythema, to itching, to possible superficial bleeding and to the infiltration of the psoriatic plaque, any adverse or undesirable effect which the volunteers may present likewise being recorded. The total volume of cream with active ingredients and of placebo cream was 14 grams for each of them.
Results: After 7 days, all the volunteers had substantially improved the desquamation of the plaques in both elbows in a similar manner, the size thereof being maintained.

After 14 and 21 days, all the volunteers did not show significant changes in any of the elbows, in relation to that observed on day 7.

In contrast, the right elbow showed in all the volunteers, less desquamation and attenuation of erythema, with a decrease of the degree of infiltration of the plaque. The itching decreased more in the right elbow than in the left elbow. Cases of bleeding were not recorded in any of the elbows.

After 28 days, all the volunteers had the same clinical characteristics on the left elbow control as in the prior evaluations. In contrast, the right elbow showed in all the volunteers, less desquamation and an attenuation of erythema, with a decrease of the degree of infiltration of the plaque.
Conclusions: The cream with 0.3% idebenone and 0.012% mimosine proved to be effective for improving the clinical appearance of psoriatic skin lesions, However, in comparison to other treatment with higher concentrations of active ingredients in the cream, the clinical improvement was not as important, and the time of onset of this improvement was substantially greater.

### Example 7

### Determination of the epidermal proliferation inhibition after the daily topical application of a cream with 0.1% mimosine and 2.5% idebenone.

For the purpose of quantifying the epidermal proliferation inhibition after the daily topical application of a cream with 0.1% mimosine and 2.5% idebenone in volunteers with psoriasis vulgaris, alternate sections of the biopsies obtained on day 28 of the treatment, described in Example 3, were subjected to enzymatic immunolabeling techniques using a monoclonal antibody (Ki67) which is a marker of cells in the proliferative stage of the cell cycle. To that end, the microtome sections were deparaffinated, hydrated by means of passages in alcohols of decreasing concentration, embedded in water and pretreated for 5 minutes with a hydrogen peroxide solution to eliminate the endogenous peroxidase activity. The sections were then washed in phosphate buffered saline (PBS) and incubated for 30 minutes in a wet chamber at room temperature, with a dilution pre-established by the manufacturer of the Ki67 monoclonal antibody (Biogenex). After two washings with PBS, the sections were treated with a commercial biotinylated antibody for another 30 minutes, used as a secondary antibody and post-treated with an antibody labeled with the peroxidase-antiperoxidase complex, according to the instructions of the manufacturer (Biogenex) and the reaction was revealed with diaminobenzidine. The sections thus treated were counter-stained with Harris Hematoxylin.
Results: The left elbow samples showed a strongly positive nuclear staining in approximately 30% of the cells of the epidermal basal layer, expressed as an average of the 10 cases evaluated, staining also being observed in cells dispersed in the spinous layer and a very small amount in cells in the most superficial epidermal. The right elbow samples showed a nuclear staining only in the epidermal basal layer in a percentage which ranged between 4% and less than 1%. No staining was observed in upper epidermal strata.
Conclusions: Given that the antigen revealed by the Ki67 antibody is a cell proliferation marker, it is concluded that the low labeling observed in the right elbow, treated with the cream with 0.1% mimosine and 2.5% idebenone, reduces the mitotic index and therefore the epidermal proliferation of psoriatic lesions to values similar to those of normal skin (to 1.5% of epidermal basal cells according to historic data). The difference in proliferation between treated psoriatic skin and psoriatic skin that is not treated or is treated with placebo is highly significant.

### Example 8

### Determination of the epidermal proliferation inhibition after the daily topical application of a cream with 2.5% idebenone in psoriatic lesions.

For the purpose of evaluating the effect of a cream with 2.5% idebenone on the epidermal proliferation of a psoriatic skin lesion, alternate sections of the biopsies obtained from both elbows of the volunteers subjected to treatment with this cream, described in Example 4, were obtained. The sections were processed in a manner identical to that described in Example 7, using the Ki67 antibody as a cell proliferation marker.
Results: The right elbow samples, treated with the combination of 0.1 % mimosine and 2.5% idebenone showed results consistently similar to those of Example 7, whereas the left elbow samples, treated with 2.5% idebenone showed a strongly positive staining in the nuclei of approximately 20% of the basal cells of the epidermal layer, with a few positive nuclei in the uppermost strata of the epidermis.
Conclusion: The cream with 2.5% idebenone shows a discrete decrease of the epidermal proliferation of psoriatic lesions when it is compared with the historic records of non-treated psoriatic skin, but the proliferation is significantly higher than that of healthy skin or of psoriatic skin treated with a cream with 0.1% mimosine and 2.5% idebenone.

### Example 9

### Determination of the epidermal proliferation inhibition after the daily topical application of a cream with 0.1% mimosine.

For the purpose of evaluating the effect of a cream with 0.1 % mimosine on the epidermal proliferation of a psoriatic skin lesion, alternate sections of the biopsies obtained from both elbows of the volunteers subjected to treatment with this cream, described in Example 4, were obtained. The sections were processed in a manner identical to that described in Example 7, using the Ki67 antibody as a cell proliferation marker.
Results: The right elbow samples, treated with the combination of 0.1% mimosine and 2.5% idebenone showed results consistently similar to those of Example 7, whereas the left elbow samples, treated with 0.1 % mimosine showed a strongly positive staining in the nuclei of approximately 5% of the basal cells of the epidermal layer, without positive staining in cells located in the uppermost strata of the epidermis.
Conclusion: The cream with 0.1% mimosine shows a marked decrease of the epidermal proliferation of psoriatic lesions when it is compared with the historic records of non-treated psoriatic skin. However, although the decrease of proliferation is not significant, it is slightly higher, on average, than that recorded in psoriatic skin treated with a cream with 0.1 % mimosine and 2.5% idebenone.

### Example 10

### Evaluation of the inflammatory lesion after the daily topical application of a cream with 0.1% mimosine and 2.5% idebenone

For the purpose of evaluating the effect of a cream with 0.1% mimosine and 2.5% idebenone on the inflammatory process of a psoriatic skin lesion, the histological sections of the biopsies obtained from both elbows of the volunteers subjected to treatment with this cream, described in Example 2, were observed, and the diameter of the vessels of the dermal papillae was analyzed morphometrically by means of image digitizing equipment (Quantimet 500+, Leica) and the number of inflammatory cells of each lesion was counted. To measure the vascular diameters, only those vessels which corresponded morphologically to capillaries, the section of which was round, were selected, the results being expressed as the average of 15 capillary vessels obtained from 5 non-contiguous 100X microscopic fields. The number of inflammatory cells, without making any distinction in the cell types forming such population, was expressed as the number of cells per dermal papillae, taking the number as the average of 10 dermal papillae arranged perpendicular to the epidermal surface, in which the contiguity with the underlying dermis could be observed, Likewise, the averages were expressed as the average of the 10 evaluated cases.
Results: The right elbow samples, treated with the combination of 0.1% mimosine and 2.5% idebenone, showed vascular diameters significantly smaller than those recorded in the left elbow skin samples, treated with a placebo cream. The average of the vascular diameters of the skin treated with the therapeutic cream was 6.5 microns, with a dispersion range from 4.3 to 8.5 microns, whereas the skin treated with placebo showed an average of 11.3 microns, with a dispersion range from 7.8 to 14.5 microns. The average of inflammatory cells per papilla was 42 cells/papilla, with a dispersion from 26 to 62 cells for the skin treated with placebo, and 23 cells/papilla, with a dispersion from 2 to 43 cells for the skin treated with the cream with 0.1 % mimosine and 2.5% idebenone.
Conclusion: The cream with 0.1% mimosine and 2.5% idebenone shows a marked decrease of the morphological parameters characterizing an inflammatory process when it is compared with those recorded in psoriatic skin treated with placebo, these results being consistent with the clinical observations and the histological symptoms.

### Example 11

### Evaluation of the inflammatory lesion after the daily topical application of a cream with 2.5% idebenone.

For the purpose of evaluating the effect of a cream with 2.5% idebenone on the inflammatory process of a psoriatic skin lesion, the histological sections of the biopsies obtained from both elbows of the volunteers subjected to treatment with this cream, described in Example 4, were observed, and the diameter of the vessels of the dermal papillae was analyzed morphometrically by means of image digitizing equipment (Quantimet 500+, Leica) and the number of inflammatory cells of each lesion was counted in a manner similar to that described in Example 10.
Results: The right elbow samples, treated with the combination of 0.1 % mimosine and 2.5% idebenone showed vascular diameters smaller than those recorded in the left elbow skin samples, treated with a cream with 2.5% idebenone. The average of the vascular diameters of the skin treated with the cream with 0.1% mimosine and 2.5% idebenone was 6.5 microns, with a dispersion range from 5.0 to 7.8 microns, whereas the skin treated with the cream with 2.5% idebenone showed an average of 6.9 microns, with a dispersion range from 5.0 to 8.2 microns. The average of inflammatory cells per papilla was 22 cells/papilla, with a dispersion from 10 to 37 cells for the skin treated with the cream with 2.5% idebenone, and 20 cells/papilla, with a dispersion from 3 to 33 cells for the skin treated with the cream with 0.1% mimosine and 2.5% idebenone
Conclusion: The cream with 0.1% mimosine and 2.5% idebenone shows a marked decrease of the morphological parameters characterizing an inflammatory process, this reduction being smaller when a cream with 2.5% idebenone is used, although this difference was not statistically significant.

### Example 12

### Evaluation of the inflammatory lesion after the daily topical application of a cream with 0.1% mimosine.

For the purpose of evaluating the effect of a cream with 0.1% mimosine on the inflammatory process of a psoriatic skin lesion, the histological sections of the biopsies obtained from both elbows of the volunteers subjected to treatment with this cream, described in Example 4, were observed, and the diameter of the vessels of the dermal papillae was analyzed morphometrically by means of image digitizing equipment (Quantimet 500+, Leica) and the number of inflammatory cells of each lesion was counted in a manner similar to that described in Example 10.
Results: The right elbow samples, treated with the combination of 0.1% mimosine and 2.5% idebenone showed vascular diameters smaller than those recorded in the left elbow skin samples, treated with a cream with 0.1% mimosine. The average of the vascular diameters of the skin treated with the cream with 0.1% mimosine and 2.5% idebenone was 6.5 microns, with a dispersion range from 5.0 to 7.8 microns, whereas the skin treated with the cream with 0.1% mimosine showed an average of 10.9 microns, with a dispersion range from 6.0 to 13.2 microns. The average of inflammatory cells per papilla was 36 cells/papilla, with a dispersion from 22 to 53 cells for the skin treated with the cream with 0.1 % mimosine, and of 20 cells/papilla, with a dispersion from 3 to 33 cells for the skin treated with the cream with 0.1 % mimosine and 2.5% idebenone.
Conclusion: The cream with 0.1% mimosine and 2.5% idebenone shows a marked decrease of the morphological parameters characterizing an inflammatory process, this reduction being smaller when a cream with 2.5% idebenone is used, although this difference is not statistically significant.

### Example 13

### Study of shelf-life stability of a formulation containing 0.012% mimosine and 0.3% idebenone.

The stability of two formulations prepared on different dates, but with an identical composition, was studied. Both were formulated in the form of an aqueous (O/W) cream containing 0.012% mimosine and 0.3% idebenone. -

The oldest formulation analyzed was packaged in a white polystyrene jar and was stored on a shelf for a period of 600 days, at room temperature and protected from light.

**Table III: Quantitative formula of an aqueous (O/W) cream containing 0.012% mimosine and 0.3% idebenone. Amounts for 100 grams.**

| COMPONENTS | % BY WEIGHT |
|---|---|
| Water | 79.7 |
| Non-ionic self-emulsifiable wax | 6.00 |
| Petroleum jelly | 5.00 |
| Glycerol | 5.00 |
| Isopropyl myristate | 2.00 |
| Cetyl alcohol | 1.50 |
| Methylparaben (Nipagin) | 0.20 |
| Propylparaben (Nipasol) | 0.10 |
| Idebenone | 0.3 |
| Mimosine | 0.012 |

The methodology used for quantifying idebenone was UV-Vis spectrophotometry, whereas the methodology used for quantifying mimosine was high performance liquid chromatography (HPLC).

A non-significant difference was obtained in the assessment of the active ingredients for the most recent creams (1 month after their preparation) and the oldest creams (20 months after their preparation). It is concluded that this cream can be considered stable for at least 600 days (20 months).

### Example 14

### Study of shelf-life stability of a formulation containing 0.1% mimosine and 2.5% idebenone

The stability of two formulations prepared on different dates, but with an identical composition, was studied. Both were formulated in the form of an aqueous (O/W) cream containing 0.1% mimosine and 2.5% idebenone.

The oldest formulation analyzed was packaged in a white polystyrene jar and was stored on a shelf for a period of 600 days, at room temperature and protected from light.

**Table V: Quantitative formula of an aqueous (O/W) cream containing 0.1% mimosine and 2.5% idebenone. Amounts for 100 grams.**

| COMPONENTS | % BY WEIGHT |
|---|---|
| Water | 79.7 |
| Non-ionic self-emulsifiable wax | 6.00 |
| Petroleum jelly | 5.00 |
| Glycerol | 5.00 |
| Isopropyl myristate | 2.00 |
| Cetyl alcohol | 1.50 |
| Methylparaben (Nipagin) | 0.20 |
| Propylparaben (Nipasol) | 0.10 |
| Idebenone | 2.50 |
| Mimosine | 0.1 |

The methodology used for quantifying idebenone was UV-Vis spectrophotometry, whereas the methodology used for quantifying mimosine was high performance liquid chromatography (HPLC).

A non-significant difference was obtained in the assessment of the active ingredient for the most recent creams (1 month after their preparation) and the oldest creams (20 months after their preparation). It is concluded that this cream can be considered stable for at least 600 days (20 months).

### Example 15

### Morphometric comparison between human psoriatic skin treated with a cream with 0.1% mimosine and 2.5% idebenone and psoriatic skin treated with a placebo cream.

The following experiment was designed for the purpose of knowing the effect of a cream with 0.1% mimosine and 2.5% idebenone on epidermal proliferative and dermal inflammatory processes of psoriatic skin.

### MATERIALS AND METHODS:

Skin samples: the histological sections of 5 volunteers of Example 5, who had received for 28 consecutive days the cream with idebenone and mimosine in the lesions of the right elbow and the placebo cream in the left elbow were used.
Morphometric study: the dermal vascular diameters of the skin treated with active ingredients and with placebo were measured by means of using a digital measurement program (Image4plus). The microcirculation vessels the morphology of which corresponded to capillaries, postcapillary venules and precapillary arterioles, the section of which was circular, were selected for the purpose of preventing distortion in the measurement. The inner vascular diameter was measured, the vessels of 10 lower-magnification microscopic fields per sample being counted. The results were expressed in microns as the average of each case.

The epidermal thickness of the skin treated with active ingredients and with placebo was measured with the same system. To that end, the distance between the most superficial cell of the granular layer and the deepest one of the epidermal basal layer was considered, measuring those thicknesses comprised between dermal papillae and discarding the suprapapillary epidermis. Ten measurements per sample were made. The results were expressed in microns as the average of each case.

### Results:

Vascular diameters: As observed in Table VI, in all the samples treated with active ingredients, a decrease of the vascular diameters in relation to the sample of the same patient treated with placebo was recorded. The reduction of the vascular diameters was 49.5% on general average, ranging between 29.41 % and 72.13%.

Epidermal thicknesses: As observed in Table VII, in all the samples treated with active ingredients, a decrease of the epidermal thickness in relation to the sample of the same patient treated with placebo was recorded. The reduction of the epidermal thickness was 33.55% on general average, ranging between a minimum of 5.97% and a maximum of 48%.

### Conclusions:

The cream with 0.1 % mimosine and 2.5% idebenone was shown to be effective in reducing the height of the epidermis of mild to moderate psoriatic patients when it is compared with a placebo cream without active ingredients applied simultaneously on the contralateral elbow of the same patient. Likewise, this cream was shown to be effective in decreasing the microcirculation vascular diameters. It is considered that the epidermal height is an indication of the proliferation of this skin layer, therefore the cream with active ingredients was shown to be effective for reducing this proliferation characterizing the disease in question. The height of the horny layer was not taken into account because it is frequent for it to become detached or break during the histological processing of the samples, giving results which could possibly be erroneous. Likewise, it is considered that the vascular diameters are a direct index of the dermal inflammatory response, therefore the cream with active ingredients was shown to be effective for reducing the inflammatory symptoms accompanying the disease in question.

**Table VI: Microcirculation vascular diameters of skin treated with a cream with mimosine (0.1%) and idebenone (2.5%) compared to those of skin treated with a placebo cream, applied on the right elbow and the left elbow, respectively, of 5 patients. The duration of the treatment was 28 days, and the measurements are expressed in microns as the average of at least 10 measurements per sample.**

| PATIENT | ELBOW | DIAMETER | DECREASE |
|---|---|---|---|
| 1 | treated | 6.7567 | 29.41% |
| 1 | placebo | 9.572 | |
| 2 | treated | 9.4982 | 32.65% |
| 2 | placebo | 14.1026 | |
| 3 | treated | 6.254075 | 39.77% |
| 3 | placebo | 9.5885 | |
| 4 | treated | 5.1477 | 72.13% |
| 4 | placebo | 18.468 | |
| 5 | treated | 8.7997 | 55.54% |
| 5 | placebo | 19.79345 | |
| AVERAGE | | | 45.90% |

**Table VII: Epidermal thicknesses of skin treated with a cream with mimosine (0.1%) and idebenone (2.5%) compared with a placebo cream applied on the right elbow and the left elbow, respectively, of 5 patients. The duration of the treatment was 28 days, and the measurements are expressed in microns as the average of at least 10 measurements per sample, taken from the granular layer to the epidermal basal layer in interpapillary areas.**

| PATIENT | ELBOW | THICKNESS | DECREASE |
|---|---|---|---|
| 1 | treated | 138.1377 | 36.90% |
| 1 | placebo | 218.919 | |
| 2 | treated | 260.1348 | 5.97% |
| 2 | placebo | 276.6511 | |
| 3 | treated | 157.0944 | 57.47% |
| 3 | placebo | 369.3685 | |
| 4 | treated | 166.7854 | 48.00% |
| 4 | placebo | 320.7578 | |
| 5 | treated | 276.4073 | 19.39% |
| 5 | placebo | 342.905 | |
| AVERAGE | | | 33.55% |

## Claims

1. A use of mimosine or a derivative thereof, **characterized in that** it is used for treating the cutaneous effects of psoriasis and related skin disorders.

2. The use of mimosine or a derivative thereof according to claim 1, **characterized in that** it is used in combination with idebenone or a derivative thereof for treating the cutaneous effects of psoriasis and related skin disorders.

3. The use of mimosine or its derivatives according to any one of claims 1 or 2, **characterized in that** it is used in a cosmetic composition intended to be applied on the skin, useful for producing a cosmetic improvement of psoriatic skin at the application site.

4. The use of mimosine or its derivatives according to any one of claims 1 or 2, **characterized in that** it is used to prepare a dermatological composition useful for producing a clinical improvement of psoriatic skin at the application site.

5. The use of mimosine or its derivatives according to any one of claims 1 or 2, **characterized in that** it is used to prepare a dermatological composition useful for producing a clinical improvement of the skin after it is applied in sites damaged by psoriasis or by related dermatological conditions.

6. The use of mimosine or its derivatives according to any one of claims 1 or 2, **characterized in that** the skin disorders are selected from the group comprising psoriasis, rosacea, photodamaged skin, atopic dermatitis, eczemas and seborrheic dermatitis.

7. The use of mimosine or its derivatives according to any one of claims 1 or 2, **characterized in that** mimosine, idebenone and/or the acceptable derivatives thereof, are found in an effective amount to produce epidermal proliferation inhibition and the clinical improvement of the skin.

8. A composition for treating the cutaneous effects of psoriasis and related skin disorders, applicable on the skin, **characterized in that** it comprises mimosine or an acceptable derivative thereof in an amount comprised between 0.01% and 30% w/w or v/w.

9. A composition for treating the cutaneous effects of psoriasis and related skin disorders, applicable on the skin according to claim 8, **characterized in that** it further comprises idebenone or an acceptable derivative thereof or an acceptable derivative thereof in an amount comprised between 0.01% and 30% w/w or v/w.

10. A composition for treating the cutaneous effects of psoriasis and related skin disorders, applicable on the skin according to any one of the previous claims, **characterized in that** it further comprises an extract of tepescohuite or of its acceptable derivatives.

11. A composition intended to be applied on the skin according to any one of the previous claims, **characterized in that** it is in the form of an occlusive patch.

12. A composition intended to be applied on the skin according to any one of claims 8 to 10, **characterized in that** it is in the form of a cream.

13. A composition intended to be applied on the skin according to any one of claims 8 to 10, **characterized in that** it is in the form of a gel.

14. A composition intended to be applied on the skin according to any one of claims 8 to 10, **characterized in that** it is in the form of an emulsion.

15. A composition intended to be applied on the skin according to any one of claims 8 to 10, **characterized in that** it is in the form of a spray.

16. A composition intended to be applied on the skin according to any one of claims 8 to 10, **characterized in that** it further comprises oily components and hydrosoluble components.

17. A composition intended to be applied on the skin according to the previous claim, **characterized in that** it comprises self-emulsifiable wax, petroleum jelly, isopropyl myristate and cetyl alcohol.

18. A composition intended to be applied on the skin according to claim 16, **characterized in that** it comprises glycerol, methylparaben and propylparaben.

19. A composition intended to be applied on the skin according to claim 16, **characterized in that** it further comprises beneficial agents for the skin.

20. A composition intended to be applied on the skin according to claim 16, **characterized in that** it further comprises cosmetically and/or pharmaceutically acceptable antioxidants.

21. A method for treating the cutaneous effects of psoriasis and related skin disorders, **characterized in that** it comprises applying at the lesioned site a composition comprising an effective amount of mimosine or its derivatives according to claims 8 or 9, allowing it to act overnight and rinsing in the morning.

22. A method for treating the cutaneous effects of psoriasis and related skin disorders, **characterized in that** it comprises applying at the lesioned site a composition comprising an effective amount of mimosine or its derivatives according to claims 8 or 9, two times a day.

23. A method for treating the cutaneous effects of psoriasis and related skin disorders according to any one of claims 21 and 22, **characterized in that** ultraviolet light radiation is further applied to the patient.
